# FASCICULE DE BREVET EUROPEEN

(11) **EP 2 674 190 B1**
(45) Date de publication et mention de la délivrance du brevet: **17.12.2014**
(21) Numéro de dépôt: 12194864.0
(22) Date de dépôt: 29.11.2012
(51) Int. Cl.: A61N 1/05, H01R 4/24

(54) **Structure d'électrode pour une microsonde multipolaire de détection/stimulation destinée à être implantée dans un vaisseau cardiaque ou cérébral**
Elektrodenstruktur für eine multipolare Detektions-/Stimulationsmikrosonde zur Implantation in ein Herz- oder Hirngefäß
Electrode structure for a multipolar stimulation/sensing microprobe for implantation in a heart or brain blood vessel

(30) Priorité: 13.06.2012 FR 1255544
(43) Date de publication de la demande: 18.12.2013
(73) Titulaire: Sorin CRM SAS, 92140 Clamart (FR)
(72) Inventeur: Ollivier, Jean-François, 91190 Villiers Le Bacle (FR); D'Hiver, Philippe, 92320 Chatillon (FR); Shan, Nicholas, 91260 Juvisy sur Orge (FR)
(74) Mandataire: Dupuis-Latour, Dominique

(56) Documents cités:
- US-A- 4 387 727
- US-A- 4 590 950
- US-A1- 2008 114 230
- US-A1- 2010 331 934
- US-B1- 7 364 479

## Description

L'invention concerne de façon générale les "dispositifs médicaux implantables actifs" tels que définis par la directive 90/385/CEE du 20 juin 1990 du Conseil des communautés européennes.

Elle concerne plus précisément une microsonde de détection/stimulation destinée à être implantée dans des réseaux veineux, artériels ou lymphatiques pour y délivrer une impulsion électrique et/ou détecter une activité électrique.

Une telle sonde peut notamment être utilisée en cardiologie, en particulier pour être implantée dans le réseau veineux du sinus coronaire afin de stimuler une cavité gauche ou droite du coeur.

Les microsondes sont cependant utiles dans de nombreuses autres applications médicales, chaque fois que l'on est en présence d'un réseau veineux, artériel ou même lymphatique, comme notamment le réseau cérébral veineux ou artériel. En particulier, la stimulation électrique a permis des avancées importantes en neurologie dans le domaine de la neuromodulation, technique qui consiste à stimuler des zones cibles du cerveau pour le traitement de troubles tels que la maladie de Parkinson, l'épilepsie et autres maladies neurologiques. Une telle technique permet une approche moins invasive de ces traitements et surtout une efficacité supérieure des traitements administrés

La difficulté dans ces applications est d'atteindre des régions peu accessibles aujourd'hui, grâce à des "microsondes" de stimulation de très petit diamètre mais présentant néanmoins une très grande robustesse afin de garantir la biostabilité à long terme.

Ainsi, la taille des sondes implantables actuelles étant typiquement de l'ordre de 4 à 6 French (1,33 à 2 mm), il serait souhaitable de pouvoir réduire ce diamètre à moins de 2 French (0,66 mm) pour ouvrir de nouvelles perspectives d'applications médicales dans des domaines jusqu'à présent non explorés compte tenu des contraintes technologiques. Une telle taille de microsonde permettrait notamment d'atteindre des veinules de très petite dimension, inaccessibles aujourd'hui avec des dispositifs de taille supérieure. Une telle microsonde doit également pouvoir naviguer facilement dans les réseaux veineux artériels ou lymphatiques en présentant une souplesse suffisante pour pouvoir être introduite dans des réseaux de vaisseaux présentant de fortes tortuosités, anastomoses, etc.

Cependant, il est clair que la réduction de diamètre des sondes augmente leur complexité technologique et impose des contraintes techniques génératrices de risques.

Il a été proposé des sondes comprenant un microcâble constitué d'un conducteur central destiné à être relié au générateur de l'implant, ce conducteur étant revêtu d'une gaine électriquement isolante. Le conducteur central est plein, le diamètre des conducteurs spiralés utilisés jusqu'à présent étant incompatible avec le diamètre requis. Ces sondes, monopolaires, présentent en outre sur leur partie active une ou plusieurs électrodes de détection/stimulation toutes électriquement reliées au conducteur central, et destinée à venir en contact avec la paroi du vaisseau-cible. Une première technique de réalisation des électrodes de telles sondes, décrite par exemple dans le EP 2 455 131 A1 (Sorin CRM SAS), consiste à dénuder ponctuellement la gaine isolante de façon à mettre à nu le microcâble en un ou plusieurs points. Les parties dénudées vont constituer ensemble un réseau d'électrodes reliées en série permettant de multiplier les points de stimulation et d'assurer ainsi une diffusion multizone de l'énergie de stimulation délivrée par l'implant.

Ce même document propose également, en variante, de réaliser la partie active de microsonde en enfilant successivement et alternativement sur le microcâble des tubes isolants et des électrodes conductrices courtes en platine iridié, en forme de bagues. Les tubes isolants, par exemple en polyuréthanne, sont collés sur le microcâble et les électrodes en platine iridié sont serties directement sur ce microcâble.

Une autre technique encore consiste à appliquer sur le microcâble une enduction de colle polyuréthanne isolante, en laissant apparaitre localement en réserve des surfaces non revêtues, conductrices.

Avec ces techniques qui prévoient la mise à nu du microcâble (ablation de l'isolant ou surfaces laissées en réserve), il est toutefois nécessaire de prévoir un revêtement conducteur, par exemple un alliage de nitrure de titane ou un dépôt de carbone de type Carbofilm (marque déposée) par une technique de pulvérisation cathodique telle que celle exposée notamment dans les US 5 370 684 A et US 5 387 247 (tous deux au nom de Sorin Biomedica SpA), pour protéger des phénomènes de corrosion le câble mis à nu.

En effet, celui-ci est réalisé en un alliage tel que le MP35N (35% Ni, 35% Co, 20% Cr et 10% Mo), considéré comme inoxydable dans des conditions standard, choix nécessité par les contraintes particulièrement sévères de robustesse mécanique et de tenue à la fatigue sur le long terme imposées au microcâble. Or, un tel matériau se révèle assez sensible à l'électrocorrosion, c'est-à-dire à un phénomène de corrosion accentué par la circulation du courant dans des zones polaires (électrodes) et par le contact avec les fluides organiques environnants (sang, etc.).

Il convient donc d'éviter tout risque de perfusion des fluides corporels vers le microcâble. Pour cette raison, le microcâble doit être parfaitement isolé de tout contact avec l'environnement de la microsonde, d'où le revêtement conducteur additionnel de NiTi ou de carbone sur les zones d'électrode ou bien le sertissage sur le microcâble de bagues en platine iridié (matériau noble, insensible à la corrosion).

Cette contrainte d'isolement du microcâble avec le milieu extérieur, notamment dans la région des électrodes, doit être respectée i) sur toute la durée de vie escomptée de la microsonde, soit dix ans, et ii) en dépit de toutes les sollicitations mécaniques que la microsonde est susceptible de subir, typiquement 400 000 000 de sollicitations en flexion sans rupture, ce chiffre correspondant au nombre moyen de battements cardiaques sur la durée de vie de la microsonde. Le respect de ces conditions est indispensable pour que la microsonde puisse être implantée de façon permanente dans le corps, typiquement dans un vaisseau coronaire.

La durée de vie est de ce fait un paramètre fondamental, qui doit être absolument pris en compte lors de la conception d'une microsonde de stimulation. En effet, les battements du coeur et le mouvement des organes induisent sur ce type de dispositif des déformations de flexion qui doivent être parfaitement maitrisées. En particulier, une microsonde de stimulation par le réseau veineux peut être le siège de déformations sous des courbures très supérieures à celles que subit une sonde conventionnelle, dans la mesure où elle doit suivre les déformations des veines. Ceci provoque des contraintes plus importantes et rend sa résistance à la fatigue plus contraignante.

Dans le cas d'une sonde monopolaire, pour obtenir cette fonction de barrière de protection, garantie dans les conditions exposées ci-dessus, du microcâble contre l'électrocorrosion au niveau des électrodes, le tout réalisé de façon industriellement simple, économique et fiable, la demande française FR 12 54548 du 16.05.2012, au nom de la Demanderesse, pour une *"Structure d'électrode pour une microsonde monopolaire de détection*/*stimulation destinée à être implantée dans un vaisseau cardiaque ou cérébral"* propose de réaliser l'électrode de détection/stimulation sous forme d'une bague métallique sertie sur le microcâble dans une région non dénudée de celui-ci. Dans la partie centrale, la face interne de la bague est en contact de liaison mécanique et électrique avec la surface du microcâble en au moins une zone de perforation de la gaine. Dans les parties d'extrémité, la face interne de la bague est en contact de liaison mécanique avec la surface de la gaine, avec interposition du matériau isolant de la gaine entre le microcâble et la bague dans des régions situées de part et d'autre de la zone de perforation de la gaine.

On comprend que l'avantage de telles électrodes est qu'elles ne nécessitent pas de dénuder le microcâble pour dégager le conducteur central plein ni de prévoir un revêtement spécifique pour protéger le microcâble du milieu extérieur, tel qu'un revêtement conducteur de NiTi ou de carbone.

Mais dans certaines applications, il est souhaitable de disposer d'une sonde multipolaire, notamment pour opérer une stimulation bipolaire par envoi d'impulsions entre deux électrodes situées en bout de sonde, ou entre deux électrodes de sondes ventriculaires droites, au lieu de le faire entre une électrode (ou série d'électrodes) et le boitier du générateur, comme avec une sonde monopolaire.

Un autre avantage des sondes multipolaires et la possibilité de mettre en oeuvre la fonction de "repositionnement électronique". Cette fonction permet à un chirurgien de sélectionner parmi les diverses électrodes présentes sur la sonde celle qui procure le meilleur compromis entre échappement au nerf phrénique, efficacité sur le plan électrique et efficacité sur le plan hémodynamique. Une telle sonde à électrodes multiples est décrite en particulier dans le EP 1 983 881 A1 (ELA Medical). De cette manière, il est possible de diriger ou rediriger le champ électrique entre différentes électrodes disposées le long de la sonde de stimulation de la cavité gauche et/ou avec une des électrodes de la sonde de stimulation de la cavité droite. Cette technologie permet de gérer les micro-déplacements et les évolutions du comportement hémodynamique (*reverse modeling*) simplement par la reprogrammation du générateur par voie télémétrique à travers la peau, sans réintervention chirurgicale lourde.

Les US 2008/0114230 A1 et US 7 364 479 B1 décrivent des structures de support d'électrode pour sonde multipolaire.

Mais, dans le cas d'une sonde multipolaire, la présence d'une pluralité de conducteurs (typiquement, trois conducteurs) courant le long du corps de sonde rend inapplicable le sertissage d'une électrode en forme de bague, qui viendrait perforer indistinctement tous les conducteurs de la sonde.

Aussi, un but de l'invention est de proposer une sonde multipolaire à microcâbles multiples qui satisfasse aux conditions suivantes :
- fiabilité de la liaison mécanique et électrique entre conducteurs et électrodes en privilégiant la continuité physique des microcâbles, afin d'éviter d'altérer le tenue mécanique immédiate des conducteurs par l'assemblage de composants de continuité électrique intermédiaires insérés entre différentes portions des microcâbles ;
- étanchéité maximale autour de la surface des microcâbles afin de ne pas les exposer aux fluides corporels ;
- diamètre extérieur typique de 1 à 2 French (0,33 à 0,66 mm) ;
- flexibilité élevée de la sonde, pour conserver une *trackabilité* maximale, essentielle dans les applications spécifiques ;
- profil isodiamètre, notamment pour faciliter l'extraction de la sonde ;
- procédure de montage en usine facile et simple.

Ce but est atteint, conformément à l'invention, grâce à une sonde de détection/stimulation multipolaire destinée à être implantée dans un réseau veineux, artériel ou lymphatique, cette sonde comprenant, de manière en elle-même connue, notamment d'après le US 2008/0114230 A1 précité : au moins deux microcâbles constitués, chacun, d'un conducteur central plein destiné à être relié à un générateur de dispositif médical implantable actif, et d'une gaine en un matériau polymère isolant entourant le conducteur central sur sa périphérie et sur sa longueur ; au moins une électrode de détection/stimulation destinée à être reliée électriquement à un conducteur central d'un microcâble et à venir contre une paroi d'un vaisseau-cible dudit réseau veineux, artériel ou lymphatique ; et des moyens de connexion électrique entre l'électrode et un conducteur central. Le diamètre externe de la sonde est au plus égal à 2 French (0,66 mm), et l'électrode comprend une bague métallique cylindrique comportant une lumière longitudinale de connexion électrique apte à recevoir un microcâble destiné à être connecté à la bague, et au moins une lumière longitudinale de traversée d'électrode.

De façon caractéristique de l'invention : la lumière longitudinale de traversée d'électrode est apte à recevoir au moins un microcâble non connecté à la bague; et l'électrode comprend également des moyens de connexion électrique par perforation comprenant au moins un tunnel de guidage transversal à la bague, ménagé entre la périphérie de la bague et la lumière de connexion électrique, et un tiroir de connexion électrique apte à coulisser dans le tunnel de guidage et muni d'une extrémité interne conformée pour perforer la gaine du microcâble présent dans la lumière de manière à réaliser un contact électrique entre la bague et le conducteur central de ce microcâble via le tiroir de connexion monté et maintenu serré dans le tunnel de guidage.

Autrement dit, la sonde selon l'invention est multipolaire, elle se présente donc avec une pluralité de microcâbles dont le nombre dépend notamment du nombre de pôles considérés, et ces microcâbles sont insérés à travers des lumières aménagées sur une pluralité de bagues métalliques disposées le long de la sonde et formant électrodes.

Chaque bague comprend une lumière de connexion recevant le microcâble dont le conducteur central doit être électriquement connecté à la bague et les autres microcâbles, qui ne doivent pas être connectés à la bague, traversent cette dernière simplement par les lumières dites de traversée d'électrode.

La connexion électrique entre le microcâble à connecter et la bague métallique est réalisée au moyen du "tiroir de connexion" dont le mouvement de coulissement à force dans le tunnel de guidage provoque la perforation de la gaine isolante du microcâble par l'extrémité interne du tiroir.

La technique de connexion électrique par perforation garantit l'étanchéité autour des microcâbles et leur protection contre l'électrocorrosion, sans qu'il soit nécessaire d'utiliser des moyens particuliers du type revêtement conducteur de NiTi ou de carbone. De plus, la continuité physique des microcâbles est assurée et confère à l'ensemble de la sonde une excellente tenue mécanique même sous courbure élevée, par exemple à la traversée de vaisseaux de forte tortuosité et d'anastomoses.

Dans un mode de réalisation préférentiel permettant une opération de perforation plus précise, l'axe de coulissement du tiroir de connexion passe par le centre de la lumière de connexion. Il est également avantageux que la sonde de l'invention comprenne deux tiroirs de connexion opposés. De plus, afin d'éviter la formation sur la périphérie de la bague métallique de protubérances qui affecteraient le profil isodiamétrique de la sonde, le tiroir de connexion en position de perforation est entièrement escamoté à l'intérieur du tunnel de guidage.

Il est possible de prévoir que la lumière de traversée d'électrode reçoive une pluralité de microcâbles non connectés à la bague, pour un encombrement limité.

Selon une caractéristique préférée de l'invention, la sonde comprend, à la périphérie de la bague, au moins une zone de rainures longitudinales non traversantes. On crée de cette manière des effets de pointe générant une meilleure transmission des impulsions électriques aux tissus.

Pour éviter des discontinuités d'isolement sur l'ensemble de la sonde, l'invention prévoit que cette dernière est recouverte d'une gaine extérieure collective en matériau isolant thermorétractable, des fenêtres étant découpées dans cette gaine autour de surfaces de détection/stimulation de la bague, qui en outre peuvent porter des rainures longitudinales.

Selon d'autres caractéristiques optionnelles avantageuses , un joint de colle souple peut être déposé aux extrémités de la bague, et un chanfrein ou bien un lamage est aménagé à chaque extrémité de bague, afin notamment de gérer au mieux le gradient de raideur bague/corps de sonde.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1a est une vue en perspective en coupe d'une bague métallique constituant une électrode d'une sonde multipolaire conforme à l'invention.
La Figure 1b montre la bague de la Figure 1a équipée de trois microcâbles.
La Figure 1c montre la bague de la Figure 1b en position de perforation d'un microcâble.
La Figure 2a est une vue de côté d'une bague métallique munie de deux chanfreins d'extrémité.
La Figure 2b est une vue en perspective de la bague de la Figure 2a.
La Figure 3 est une vue éclatée de la bague des Figures 2a et 2b.
La Figure 4a est une vue en perspective d'un tiroir de connexion.
La Figure 4b montre le tiroir de connexion de la Figure 4a en position dans un tunnel de guidage.
La Figure 5 est une vue en perspective d'une pluralité de bagues métalliques constituant les électrodes d'une sonde multipolaire.
La Figure 6 représente des grappes de bagues métalliques disposées de manière à réaliser des sondes multipolaires conformes à l'invention.
La Figure 7a est une vue en perspective en coupe d'une bague métallique portant des rainures longitudinales.
La Figure 7b est une vue en perspective de la bague de la Figure 7a.
La Figure 8a est une vue en perspective d'un élément de sonde multipolaire entourée d'une gaine collective thermorétractable.
La Figure 8b montre l'élément de sonde de la Figure 8a après chauffage de la gaine thermorétractable.
La Figure 8c montre l'élément de sonde de la Figure 8b munie de surfaces de détection/stimulation.
La figure 9a est une vue en perspective éclatée d'une bague munie d'un joint de colle.
La figure 9b est une vue de détail de la Figure 9a.
La Figure 10a est une vue en perspective éclatée d'une bague munie d'un lamage d'extrémité.
La Figure 10b montre la bague de la Figure 10a équipée de microcâbles.
Les Figures 11a à 12b sont des vues en coupe de variantes de réalisation de la bague métallique.
Les Figures 13a à 13c et 14a à 14b illustrent encore deux autres variantes respectives de réalisation de la bague métallique.

On va maintenant décrire un exemple de réalisation des électrodes de la sonde multipolaire selon l'invention.

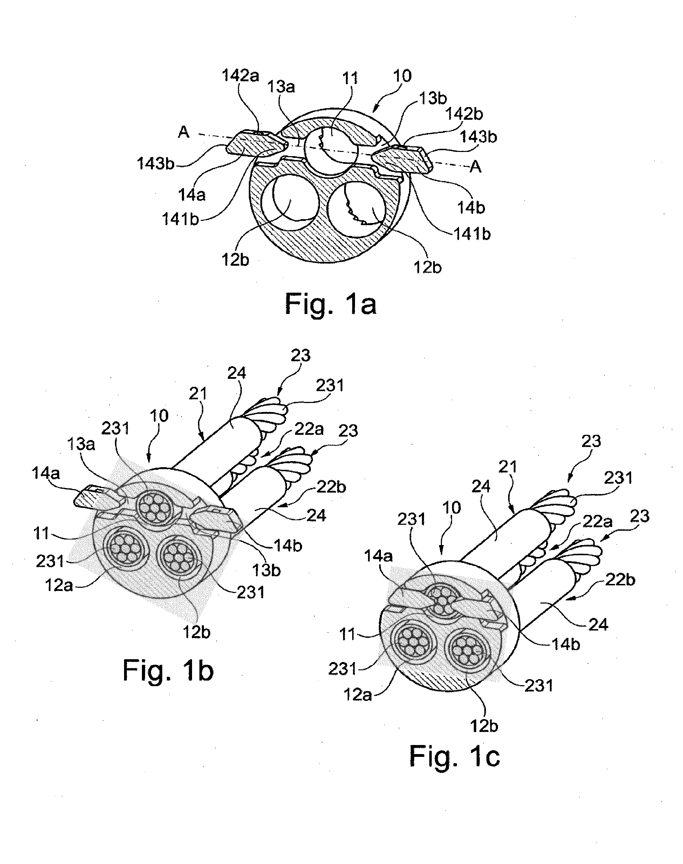

Sur les Figures 1a, 1b et 1c est représentée une bague métallique cylindrique 10 constituant une électrode de détection/stimulation pour une sonde multipolaire qui, dans l'exemple de réalisation proposé, compte trois microcâbles respectivement référencés 21, 22a et 22b (en pratique, le nombre de microcâbles varie généralement entre deux et quatre). Cette électrode est destinée à être électriquement reliée à l'un des microcâbles et à venir en contact avec une paroi d'un vaisseau-cible du réseau veineux, artériel ou lymphatique.

La bague 10 est en un matériau insensible au phénomène d'électrocorrosion tel qu'un alliage de platine iridié (typiquement 90/10) ou d'un autre métal noble, comme le palladium ou le tantale.

Pour obtenir une sonde fiable, il est proposé d'adopter la technique du câblage individualisé, chaque électrode ou groupe d'électrodes étant reliée(s) au même pôle du générateur implanté, c'est-à-dire chaque bague ou groupe de bagues étant connectée(s) à un microcâble lui-même relié au pôle considéré, l'ensemble de la structure formant une sonde de type monocorps.

La difficulté technique est alors d'établir une connexion fiabilisée industrialisable avec celui des microcâbles qui constitue la ligne de conduction, tout en préservant l'isolement des autres microcâbles non connectés, le tout devant être contenu dans un diamètre de 1 à 2 French (0,33 à 0,66 mm) pour préserver les bénéfices de tels dispositifs, par exemple le passage d'anastomoses pour établir un système stimulant du ventricule gauche via deux zones distinctes.

La solution proposée, telle qu'illustrée sur les Figures 1a, 1b et 1c, consiste en ce que la bague métallique cylindrique 10, de diamètre sensiblement égal à 1 à 2 French (0,33 à 0,66 mm), comporte trois lumières longitudinales 11, 12a et 12b s'étendant parallèlement à l'axe de la bague 10.

Plus précisément, une de ces lumières, la lumière 11 dite "de connexion électrique", reçoit le microcâble 21 prévu pour être connecté à la bague 10 et à un pôle du générateur.

Le microcâble est normalement un microcâble non dénudé, pour bénéficier aux mieux de tous les avantages des l'invention, mais en variante il peut être dénudé localement, les moyens que l'on va décrire étant alors destinés à réaliser la prise de contact directement sur la région dénudée du câble, sans perforation de l'isolant.

Les deux autres lumières longitudinales 12a et 12b sont dites "de traversée d'électrode" dans la mesure où elles permettent aux microcâbles 22a et 22b, qui ne doivent pas être connectés à la bague 10, de seulement la traverser.

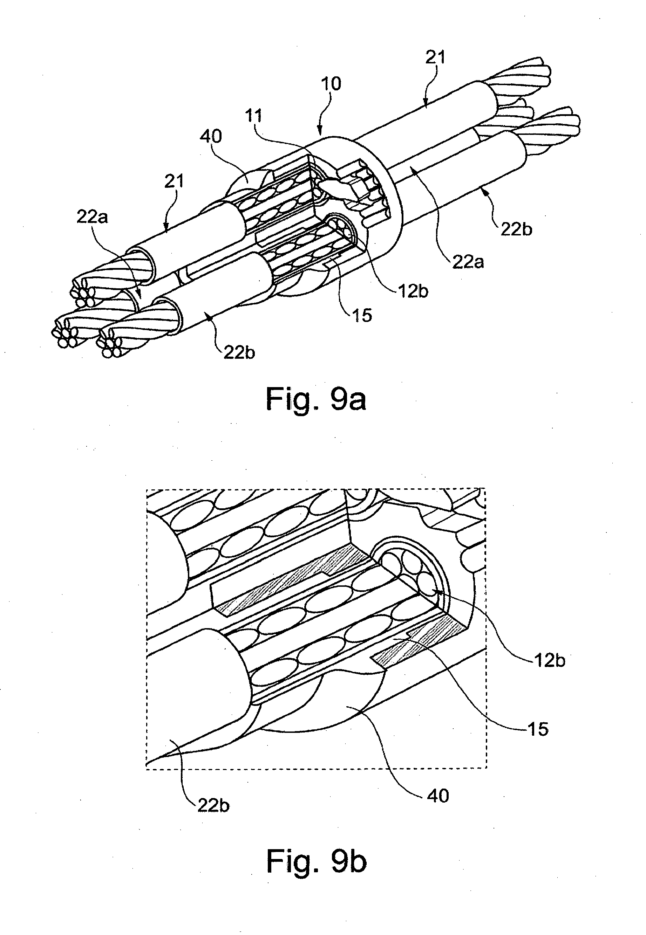

La lumière de connexion 11 présente une ouverture circulaire de diamètre sensiblement égal à celui du microcâble connecté 21 (moyennant un jeu minimum de montage, lié aux tolérances dimensionnelles des composants). À l'inverse, les lumières de traversée d'électrode 12a et 12b peuvent avoir un diamètre supérieur à celui des microcâbles non connectés 22a et 22b, ceci afin d'éviter tout risque d'endommagement de l'isolant des microcâbles à proximité immédiate de la bague ; cette augmentation de diamètre permet également d'insérer dans les lumières de traversée d'électrode une gaine isolante supplémentaire de protection (non représentée), permettant en outre, du point de vue mécanique, de contribuer à la gestion des gradients de raideur à proximité immédiate de la bague. On peut toutefois également prévoir, dans une zone centrale longitudinale de la bague 10, un rétrécissement des lumières de traversée d'électrode 12a, 12b à un diamètre sensiblement égal à celui de la lumière de connexion 11, de manière à garantir le centrage des microcâbles non connectés 22a et 22b. La zone 15 de plus fort diamètre est montrée plus particulièrement sur la vue de détail de la Figure 9b.

Il est également possible de remplacer les lumières de traversée d'électrode individuelles 12a et 12b par une seule lumière (non représentée) apte à recevoir une pluralité de microcâbles non connectés. Cette lumière unique peut par exemple avoir une forme oblongue courbée. Dans le mode de réalisation présenté, trois lignes de conduction indépendantes, portées par les microcâbles 21, 22a et 22b, ont été prises en compte afin d'optimiser le rapport diamètre hors tout/nombre de lignes. Néanmoins, une approche similaire est envisageable pour deux à quatre lignes indépendantes ou redondantes, deux lignes étant alors affectées à un groupe d'électrodes.

Pour la version illustrée à trois lignes, il est possible d'attribuer différentes fonctions spécifiques au troisième microcâble lorsque le générateur ne dispose que d'un port de connexion bipolaire (connexion de typelS1) :
- redondance de la connexion avec une série d'électrodes ;
- radio-opacité au moyen d'une section de brins en matériau radioopaque, par exemple majoritairement en Ptlr ;
- résistance mécanique pure en traction et en flexion, grâce à une section par exemple majoritairement en MP35 NLT.

Les microcâbles 21, 22a et 22b sont formés d'un conducteur central plein 23 entouré d'une gaine 24 en matériau polymère isolant.

Le conducteur central 23 est avantageusement une structure multifilaire se présentant sous forme d'un toron d'une pluralité de brins conducteurs 231 de faible diamètre. Typiquement, un toron comporte sept brins 231 de 33 µm de diamètre pour un diamètre total de 100 µm. Selon un exemple de réalisation particulier, un brin 231 présente une structure bi-matériau, à savoir une âme en platine et une enveloppe inoxydable en un alliage du type MP35 N (35% Ni, 35% Co, 20% Cr et 10% Mo) ou MP35 NLT, dont l'avantage essentiel est son extrême endurance en fatigue. La réduction en diamètre des brins unitaires permet de réduire la contrainte appliquée à chacun d'entre eux, et donc d'augmenter les performances en fatigue de la structure du toron. Une telle structure, sans lumière interne et avec plusieurs brins torsadés ensemble, est capable d'assurer à la fois l'endurance à l'encontre des mouvements cardiaques et la résistance aux sollicitations liées à la pose.

Si besoin, à titre de précaution supplémentaire, le MP35N peut être revêtu de platine afin de garantir une protection complète de la jonction électrique à l'encontre de l'électrocorrosion, au cas où l'étanchéité au niveau de la ponction à travers l'isolant, ou entre le tiroir de connexion et le tunnel de guidage, ou même l'étanchéité du collage éventuel, ne seraient pas suffisantes.

En ce qui concerne la gaine isolante 24, celle-ci doit répondre aux caractéristiques suivantes : résistance à la fatigue, isolement électrique, bio-compatibilité à long terme, biostabilité, possibilité de transformation et compatibilité avec le conducteur central 23.

Les matériaux pouvant être utilisés dans ce cadre sont notamment les matériaux du groupe comprenant : les polyuréthannes (PU), les polyesters (PET), les polyamides (PA), les polycarbonates (PC), les polyimides, les polymères fluorés, le polyéther-éther-kétone (PEEK), le poly-p-xylylène (parylène) et le polyméthacrylate de méthyle (PMM).

Cependant, on privilégiera les matériaux à forte inertie chimique comme les polymères fluorés, qui présentent également une très bonne qualité d'isolement. Parmi ces composés, on peut notamment citer les matériaux du groupe comprenant : le PTFE (polytétrafluoroéthylène), le FEP (propylène perfluoré), le PFA (résine de copolymère perfluoroalkoxy), le THV (tétrafluoroéthylène, hexafluoropropylène, fluorure de vinylidène), le PVDF (polyfluorure de vinylidène), l'EFEP (éthylène propylène éthylène fluoré) et l'ETFE (éthylène tétrafluoroéthylène).

Les procédés de réalisation de la gaine 24 d'isolement sur le conducteur central 23 sont fonction des matériaux utilisés, par exemple : co-extrusion sur le conducteur (pour PU, PA, PEEK, polyimides et polymères fluorés) ; dépôt par trempage dans une solution (pour PU, PA et polyimides) ; échauffement d'un tube thermorétractable (pour PET et polymères fluorés) ; dépôt chimique par voie gazeuse (pour le parylène) ; traitements plasma pour améliorer l'adhésion entre les couches.

On notera par ailleurs que, bien que l'invention ait été illustrée avec une unique couche d'un même matériau gainant les microcâbles 21, 22a et 22b, il est possible de prévoir plusieurs couches successives formant la gaine 24, par exemple une couche de PET et une couche de ETFE. Dans l'application proposée ici, la gaine isolante 24 a une épaisseur typique de 5 à 20 µm.

Comme le montrent les Figures 1a, 1b et 1c, la connexion électrique entre la bague 10 et le microcâble 21 est réalisée par perforation de la gaine isolante 24 du microcâble au moyen de deux tiroirs de connexion métalliques transverses 14a, 14b, chacun muni d'une extrémité interne 141 a, 141b conformée pour perforer la gaine du microcâble 21 présent dans la lumière de connexion 11, de manière à réaliser un contact électrique entre la bague 10 et le conducteur central 23 du microcâble 21 via les tiroirs 14a, 14b.

Plus précisément, les tiroirs de connexion comportent une extrémité interne 141 a, 141 b de forme saillante et peuvent coulisser respectivement dans des tunnels de guidage 13a, 13b transversaux à la bague 10, aménagés entre la périphérie de la bague et la lumière de connexion 11.

Pour éviter qu'ils ne forment des protubérances à l'extérieur de la périphérie de la bague 10, les tiroirs de connexion 14a, 14b en position de perforation sont de préférence escamotés à l'intérieur des tunnels de guidage 13a, 13b respectifs.

L'ajustement entre un tiroir de connexion et le tunnel de guidage associé est prévu suffisamment serré afin :
- d'établir la liaison électrique entre le tiroir et la bague 10 ;
- de maintenir le tiroir en position fermée. Sur ce point, il est possible d'aménager des bossages 142a, 142b sur les tiroirs 14a, 14b afin de localiser et mieux maitriser l'interférence mécanique ; et
- d'empêcher l'infiltration de fluides corporels vers les brins du microcâble 21, afin de prévenir les risques de corrosion.

En pratique, la perforation effective de la gaine isolante 24 du microcâble 21 à connecter est réalisée par enfoncement à force au moyen de mors de sertissage venant en appui sur des surfaces 143a et 143b de poussée des tiroirs de connexion 14a, 14b.

Le système à deux tiroirs de connexion opposés permet de fiabiliser la connexion microcâble/bague, d'une part, par un "effet tenaille" sur le microcâble recevant des pressions de connexion opposées et, d'autre part, par redondance du contact sur le microcâble.

Idéalement, les tiroirs de connexion opposés 14a, 14b coulissent dans les tunnels 13a, 13b selon un même axe A-A de coulissement passant par le centre de la lumière de connexion 11, afin d'orienter les surfaces de poussée 143a, 143b perpendiculairement au mouvement des mors de sertissage. Une solution alternative consiste à rendre parallèles les surfaces de poussée des tiroirs de connexion, permettant d'utiliser un équipement de sertissage à mors parallèles et non concentriques.

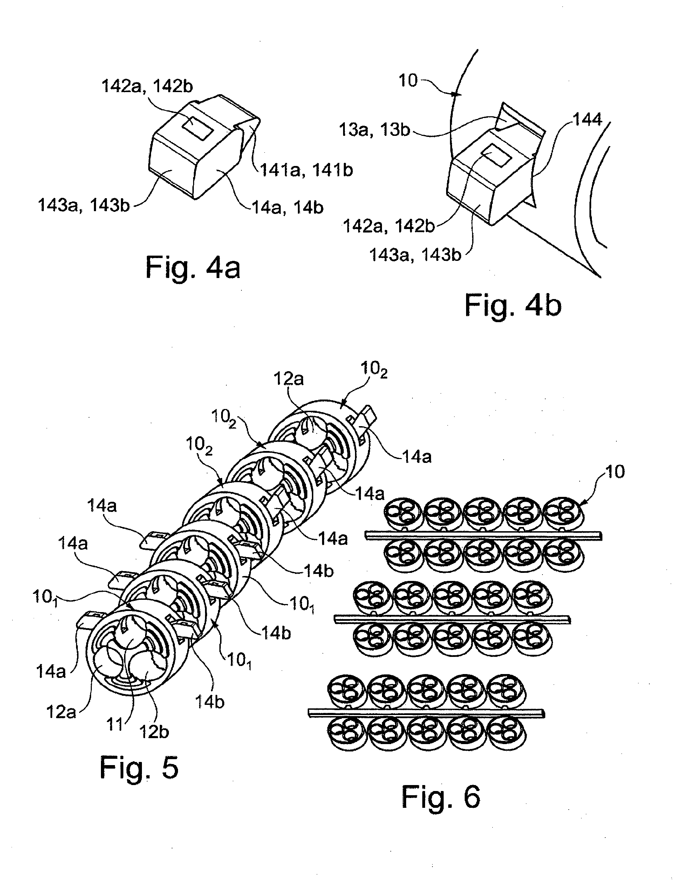

La Figure 5 montre un élément de sonde multipolaire comprenant six bagues, les trois premières 10₁ sont connectées à un même microcâble, tandis que les trois dernières 10₂ sont connectées à un autre microcâble du fait d'une orientation différente des bagues. Le choix du microcâble connecté pour une électrode donnée est fixé par le positionnement angulaire de la bague lors du pré-assemblage de la sonde dans son ensemble. Afin d'éviter tout risque d'erreur au montage, la séquence angulaire d'empilement des bagues peut être encodée sur l'outil de montage, le détrompage étant obtenu du fait que la zone de protubérance des tiroirs de connexion 14a, 14b doit entrer dans une encoche aménagée sur l'outil.

La solution alternative de la Figure 6 consiste à empiler des grappes de bagues les une au-dessus des autres, la position de chaque bague 10 sur une grappe étant déterminée par le choix du microcâble à connecter. L'alignement des lumières permet l'introduction simultanée en une seule étape des microcâbles à travers toutes les bagues, ces dernières étant ensuite détachées de la grappe. Il reste alors à positionner longitudinalement les bagues avant l'opération de sertissage.

Lors de l'insertion des microcâbles, les extrémités internes saillantes 141 a, 141 b de perforation des tiroirs de connexion 14a, 14b sont complètement escamotées à l'intérieur des tunnels de guidage 13a, 13b, évitant ainsi toute difficulté d'introduction des microcâbles puisque les lumières de connexion 11 sont alors totalement dégagées.

Un ou plusieurs zones d'attache temporaire, telle que la zone 144 de la Figure 4b, sont prévues dans la direction axiale par exemple. Ces zones sont destinées à maintenir les tiroirs 14a, 14b en position ouverte pendant toutes les opérations de fabrication des bagues, de contrôle, et d'assemblage des câbles jusqu'au stade de sertissage. Sous l'effort des mors, les zones d'attache temporaires sont rompues et n'entravent plus le déplacement de coulissement des tiroirs dans les tunnels 13a, 13b de guidage.

On peut remarquer que l'opération de sertissage concentrique, maitrisé en déplacement, ne nécessite pas de repérage angulaire de l'ensemble. De plus, une inspection visuelle rapide de production et/ou de contrôle final permet d'identifier l'état du système, à savoir état "connecté" pour des électrodes sans protubérance, ou état "non connecté" pour des électrodes avec une ou deux protubérances.

Un procédé de réalisation préférentiel de réalisation des bagues 10 est le procédé *MICA Freeform* (marque déposée de la société Microfabrica, Inc.) pour ses spécificités : extrême précision, intégration des composants, etc. Grâce à cette technologie par strates successives, la bague métallique 10, les tiroirs de connexion 14a, 14b et les zones 144 d'attache temporaire ne forment qu'une seule pièce monobloc.

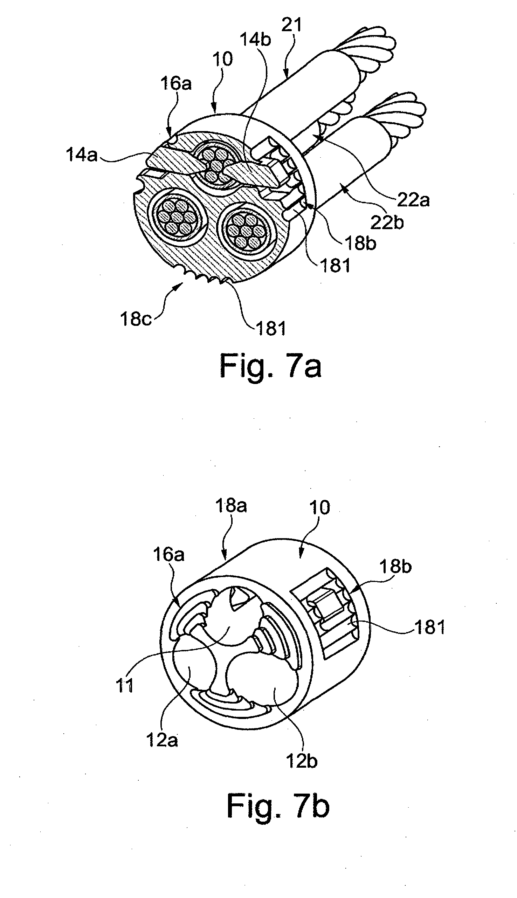

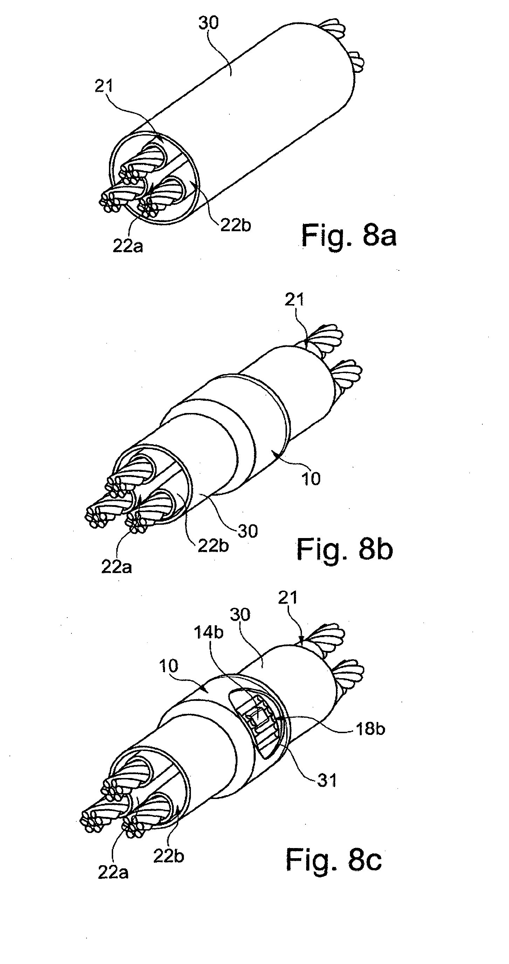

Dans le but d'améliorer par effet de pointe la transmission des impulsions électriques aux tissus visés, il est prévu sur la périphérie de la bague 10 des zones, référencées 18a, 18b et 18c sur les Figures 7a et 7b, portant des rainures longitudinales 181 affleurantes contenues à l'intérieur de la surface de la bague. Ces rainures 181, d'un rayon au moins égal à 5 µm, ne sont pas traversantes pour maintenir à chaque extrémité de la bague 10 un périmètre cylindrique qui garantisse la fonction d'étanchéité longitudinale de l'électrode lors de la pose d'une gaine extérieure collective thermorétractable 30 montrée aux Figures 8a, 8b et 8c.

Cette gaine collective 30 évite toute discontinuité de l'isolement global et la création de gradients de raideur locaux. Des fenêtres 31 sont découpées dans la gaine collective 30 par ablation laser autour de surfaces de stimulation, à savoir ici les zones 18a, 18b et 18c de rainurage longitudinal. Seules ces zones de rainurage en contact avec les tissus sont stimulantes.

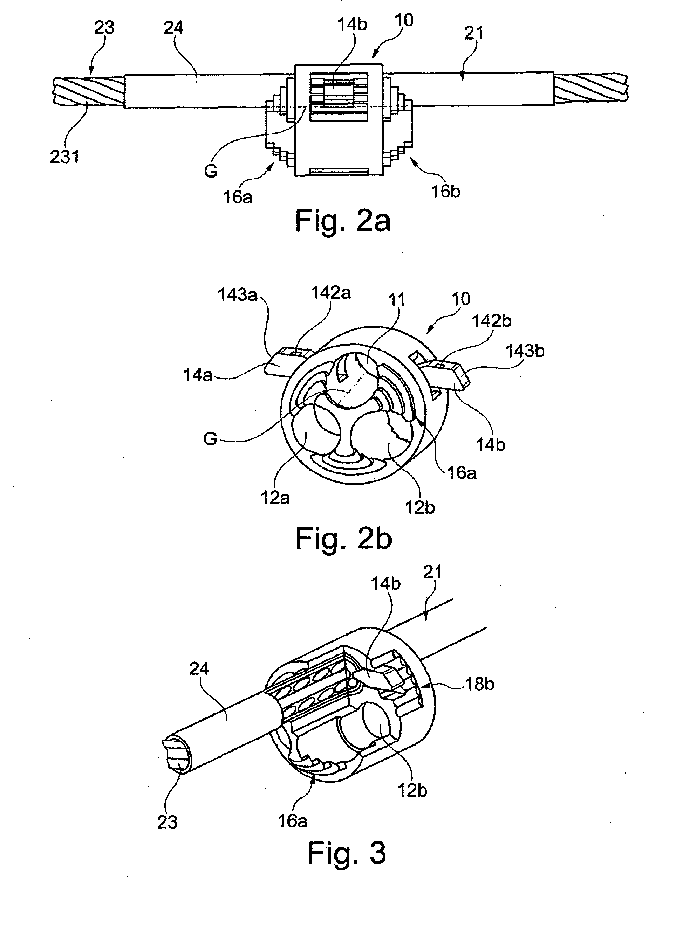

Sur les Figures 2a, 2b et 3 est représentée une bague métallique 10 munie à chaque extrémité d'un chanfrein 16a, 16b. Cette caractéristique présente l'avantage de protéger les gaines isolantes 24 des microcâbles d'un contact abrasif avec le bord externe de la bague. En effet, sous l'action de la gaine thermorétractable 30, les trois microcâbles peuvent être maintenus en position dans leur lumière respective sur une longueur plus grande correspondant à la génératrice G de la lumière de connexion 11 montrée sur les Figures 2a et 2b. Ces chanfreins permettent en outre de gérer au mieux le gradient de raideur bague/corps de sonde.

Les chanfreins 16a, 16b sont réalisés en même temps que la bague 10 selon la technologie *MICA Freeform,* ce qui leur confère le profil en escalier que l'on peut observer sur les figures précitées.

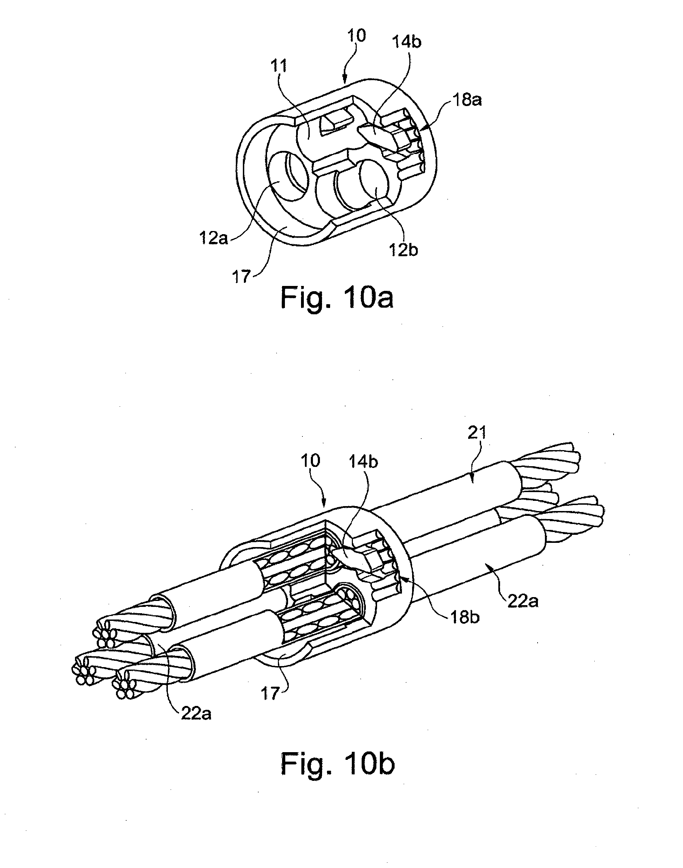

Afin de permettre aux microcâbles de venir en appui contre une surface rigide le long d'une de leur génératrice extérieure, il est possible, comme le montrent les Figures 10a et 10b, d'aménager un lamage 17 commun à chaque extrémité de la bague 10. De préférence, le lamage 17 est tangent aux trois lumières 11, 12a et 12b. On évite ainsi un contact trop ponctuel contre le rebord de la bague 10, qui risquerait de concentrer les contraintes en un point de la gaine isolante 24 des microcâbles et affaiblir localement la gaine.

Le gradient de rigidité et l'étanchéité de la structure peuvent encore être améliorés par l'ajout d'un joint de colle souple 40 aux extrémités de la bague 10, sous la gaine collective, comme représenté sur les Figures 9a et 9b. Ce joint souple 40 s'intercale entre les bords de la bague 10 et la gaine d'isolement des microcâbles non connectés 22a, 22b, dans la zone 15 de plus fort diamètre des lumières de traversée d'électrode 12a, 12b. Une colle de type polyuréthanne est particulièrement bien adaptée, du fait de sa grande fluidité et de sa capacité à migrer dans les faibles interstices par simple capillarité.

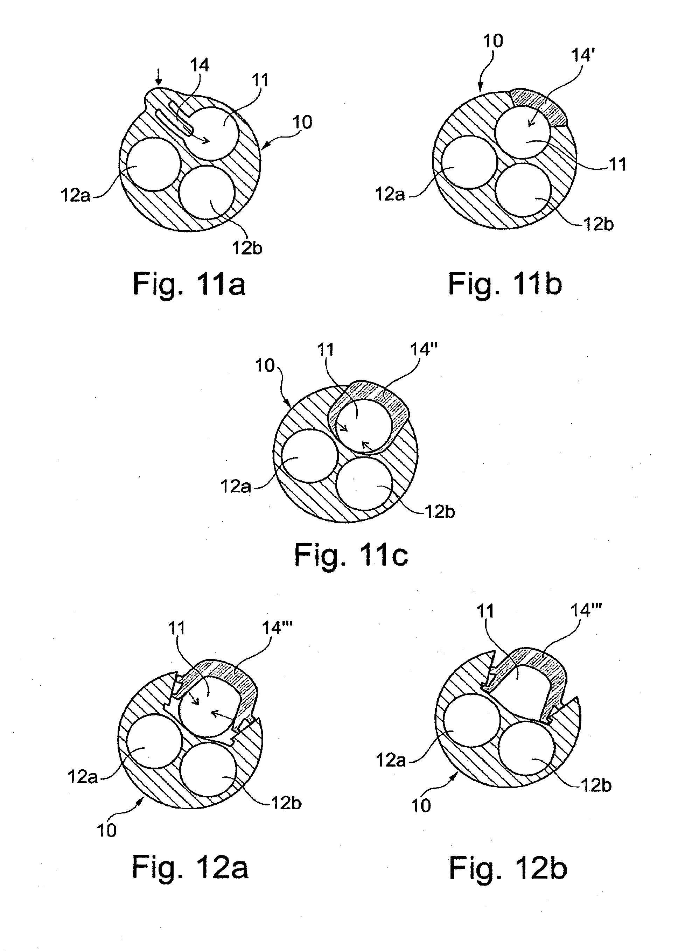

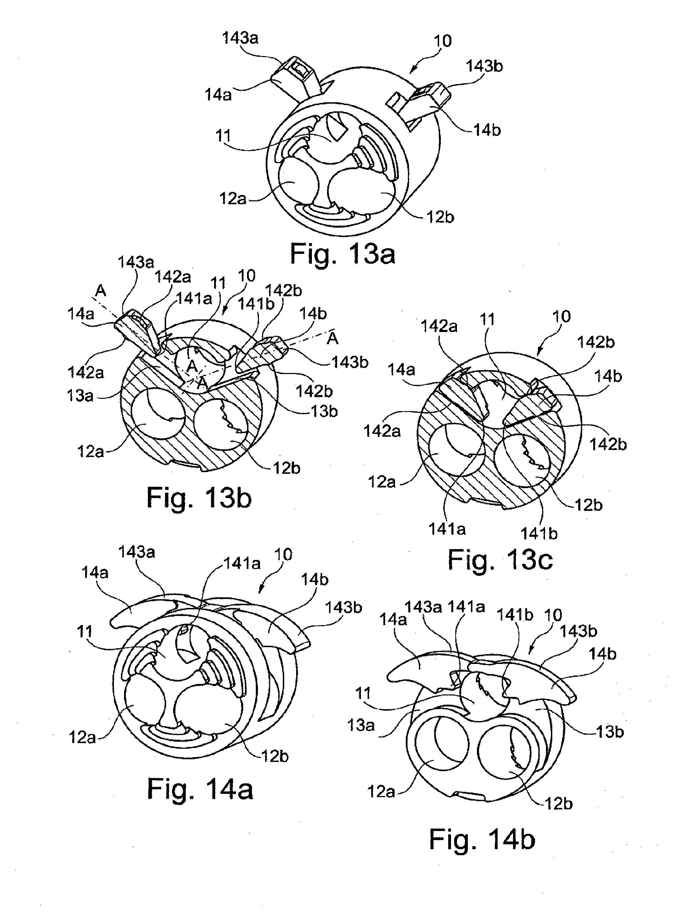

Les Figures 11 a à 12b sont des vues de côté en coupe de variantes de réalisation de bagues métalliques :
- la variante de la Figure 11a met en oeuvre un poinçon 14 de connexion découpé dans la bague 10, apte à perforer la gaine d'un microcâble disposé dans la lumière de connexion 11 par déformation maitrisée de la structure circulaire de la bague sans rupture des points d'attache du poinçon 14 avec la bague. L'avantage de cette variante est la continuité de matière dans la liaison électrique poinçon/bague et le maintien d'une étanchéité optimale ;
- sur la figure 11b, la bague 10 et l'organe 14' de perforation sont constitués de deux matériaux de rigidités différentes, combinés dans la même section de matriçage afin de programmer la déformation localisée de la lumière de connexion 11. Cette variante ne nécessite pas de repérage angulaire pendant le sertissage ;
- les variantes des Figures 11c à 12b présentent des éléments 14", 14"' de connexion mobiles uniques de type fourche, dont les avantages sont une double découpe de la gaine isolante du microcâble à connecter et un effet de "tenaille" natif. Plus particulièrement, le mode de réalisation des Figures 12a et 12b comporte un système de clipsage de l'élément 14"' de connexion dans la lumière de connexion 11 ;
- la variante des Figures 13a à 13c illustre une alternative au mouvement de translation des tiroirs 14a, 14b selon un axe A-A passant par le centre de la lumière de connexion 11 ;
- la variante des Figures 14a à 14b illustre une alternative mettant en oeuvre une rotation des tiroirs de connexion 14a, 14 dans leur tunnel de guidage 13a, 13b.

## Revendications

1. Une sonde de détection/stimulation multipolaire destinée à être implantée dans un réseau veineux, artériel ou lymphatique, comprenant :
- au moins deux microcâbles (21, 22a, 22b) constitués, chacun, d'un conducteur central plein (23) destiné à être relié à un générateur de dispositif médical implantable actif, et d'une gaine (24) en un matériau polymère isolant entourant le conducteur central sur sa périphérie et sur sa longueur ;
- au moins une électrode de détection/stimulation destinée à être reliée électriquement à un conducteur central (23) d'un microcâble (21) et à venir contre une paroi d'un vaisseau-cible dudit réseau veineux, artériel ou lymphatique ; et
- des moyens de connexion électrique entre l'électrode et un conducteur central,
et dans laquelle :
- le diamètre externe de la sonde est au plus égal à 2 French (0,66 mm) ; et
- ladite au moins une électrode comprend une bague métallique cylindrique (10) comportant une lumière longitudinale (11) de connexion électrique apte à recevoir un microcâble (21) destiné à être connecté à la bague, et au moins une lumière longitudinale (12a, 12b) de traversée d'électrode,
**caractérisée en ce que** :
- la lumière longitudinale (12a, 12b) de traversée d'électrode est apte à recevoir au moins un microcâble (22a, 22b) non connecté à la bague ; et
- ladite au moins une électrode comprend également des moyens de connexion électrique par perforation comprenant au moins un tunnel de guidage (13a, 13b) transversal à la bague, ménagé entre la périphérie de la bague et la lumière de connexion électrique, et un tiroir de connexion électrique (14a, 14b) apte à coulisser dans le tunnel de guidage et muni d'une extrémité interne (141 a, 141 b) conformée pour perforer la gaine (24) du microcâble présent dans la lumière de connexion de manière à réaliser un contact électrique entre la bague et le conducteur central de ce microcâble via le tiroir de connexion monté et maintenu serré dans le tunnel de guidage.

2. La sonde de la revendication 1, dans laquelle l'axe (AA) de coulissement du tiroir de connexion passe par le centre de la lumière de connexion.

3. La sonde de la revendication 1, comprenant deux tiroirs de connexion opposés.

4. La sonde de la revendication 1, dans laquelle le tiroir de connexion en position de perforation est entièrement escamoté à l'intérieur du tunnel de guidage.

5. La sonde de la revendication 1, dans laquelle l'extrémité interne du tiroir de connexion a une forme saillante.

6. La sonde de la revendication 1, dans laquelle la lumière de connexion présente une ouverture circulaire de diamètre égal au diamètre du microcâble connecté à la bague.

7. La sonde de la revendication 1, dans laquelle ladite au moins une lumière de traversée d'électrode présente une ouverture circulaire de diamètre supérieur au diamètre du microcâble non connecté à la bague.

8. La sonde de la revendication 7, comprenant une gaine isolante de protection supplémentaire insérée dans ladite au moins une lumière de traversée d'électrode.

9. La sonde de la revendication 1, dans laquelle l'ouverture circulaire de ladite au moins une lumière de traversée d'électrode présente, dans une zone centrale de la bague, un diamètre égal au diamètre de l'ouverture circulaire de la lumière de connexion.

10. La sonde de la revendication 1, dans laquelle ladite au moins une lumière de traversée d'électrode est apte à recevoir une pluralité de microcâbles non connectés à la bague.

11. La sonde de la revendication 1, comprenant au moins une zone (144) d'attache temporaire ente le tiroir de connexion et la bague (10).

12. La sonde de la revendication 1, comprenant, à la périphérie de la bague, au moins une zone (18a, 18b, 18c) de rainures longitudinales (181) non traversantes.

13. La sonde de la revendication 12, dans laquelle le rayon des rainures longitudinales est au moins égal à 5 µm.

14. La sonde de la revendication 1, dans laquelle ladite sonde est recouverte d'une gaine extérieure collective (30) en matériau isolant thermorétractable, des fenêtres (31) étant découpées dans cette gaine autour de surfaces (18a, 18b, 18c) de détection/stimulation de la bague.

15. La sonde de la revendication 14, dans laquelle les surfaces de détection/stimulation sont des zones portant des rainures longitudinales (181).

16. La sonde de la revendication 1, dans laquelle un joint de colle souple (40) est déposé aux extrémités de bague.

17. La sonde de la revendication 16, dans laquelle la colle souple est en polyuréthanne.

18. La sonde de la revendication 1, dans laquelle un chanfrein (16a, 16b) est aménagé à chaque extrémité de bague.

19. La sonde de la revendication 1, dans laquelle un lamage (17) est aménagé à chaque extrémité de bague.

20. La sonde de la revendication 19, dans laquelle le lamage est tangent aux lumières de la bague.

21. La sonde de la revendication 1, dans laquelle les moyens de connexion électrique comprennent un poinçon (14) de connexion découpé dans la bague.

22. La sonde de la revendication 1, dans laquelle les moyens de connexion électrique comprennent un organe (14') de perforation combiné à la bague dans une même section de matriçage, l'organe de perforation et la bague étant constitués de deux matériaux de rigidité différente.

23. La sonde de la revendication 1, dans laquelle les moyens de connexion électrique comprennent un élément (14" ; 14"') de connexion mobile unique de type fourche.

24. La sonde de la revendication 23, dans laquelle l'élément (14"') de connexion comporte un système de clipsage dans la lumière de connexion.

## Patentansprüche

1. Multipolare Detektions-/Stimulationssonde zur Implantation in einem arteriellen oder lymphatischen Venennetz, die Folgendes aufweist:
- mindestens zwei Mikrokabel (21, 22a, 22b), die jeweils aus einem massiven zentralen Leiter (23), der dazu bestimmt ist, mit einem Generator einer aktiven implantierbaren medizinischen Vorrichtung verbunden zu sein, und einer Hülle (24) aus einem isolierenden Polymermaterial bestehen, die den zentralen Leiter auf seinem Umfang und auf seiner Länge umgibt,
- mindestens eine Detektions-/Stimulations-elektrode, die dazu bestimmt ist, elektrisch mit einem zentralen Leiter (23) eines Mikrokabels (21) verbunden zu sein und gegen eine Wand eines Zielgefäßes des arteriellen oder lymphatischen Venennetzes zu kommen, und
- Mittel zum elektrischen Verbinden zwischen der Elektrode und einem zentralen Leiter,
und bei der:
- der Außendurchmesser der Sonde maximal 2 French (0,66 mm) beträgt, und
- die mindestens eine Elektrode einen zylindrischen Metallring (10) aufweist, der ein Längsloch (11) zum elektrischen Verbinden aufweist, das geeignet ist, ein Mikrokabel (21) aufzunehmen, das dazu bestimmt ist, mit dem Ring verbunden zu sein, und mindestens ein Lumen (12a, 12b) zum Durchqueren der Elektrode,
**dadurch gekennzeichnet, dass**
- das Lumen (12a, 12b) zum Durchqueren der Elektrode geeignet ist, mindestens ein Mikrokabel (22a, 22b), das nicht mit dem Ring verbunden ist, aufzunehmen, und
- die mindestens eine Elektrode auch Mittel zum elektrischen Verbinden durch Perforierung aufweist, die mindestens einen Führungstunnel (13a, 13b) quer zu dem Ring, der zwischen dem Umfang des Rings und dem Lumen zum elektrischen Verbinden eingerichtet ist, und einen Schieber zum elektrischen Verbinden (14a, 14b) aufweisen, der geeignet ist, in dem Führungstunnel zu gleiten und mit einem internen Ende (141a, 141b) versehen ist, das ausgebildet ist, um die Hülle (24) des Mikrokabels, das in dem Verbindungslumen vorliegt, zu perforieren, so dass ein elektrischer Kontakt zwischen dem Ring und dem zentralen Leiter dieses Mikrokabels über den installierten Verbindungsschieber hergestellt und in dem Führungstunnel eng gehalten wird.

2. Sonde nach Anspruch 1, bei der die Achse (AA) zum Gleiten des Verbindungsschiebers durch die Mitte des Verbindungslumens verläuft.

3. Sonde nach Anspruch 1, die zwei einander gegenüberliegende Verbindungsschieber aufweist.

4. Sonde nach Anspruch 1, bei der der Verbindungsschieber in Perforierungsposition vollständig in das Innere des Führungstunnels eingezogen ist.

5. Sonde nach Anspruch 1, bei der das interne Ende des Verbindungsschiebers eine vorstehende Form hat.

6. Sonde nach Anspruch 1, bei der das Verbindungslumen eine kreisförmige Öffnung mit einem Durchmesser gleich dem Durchmesser des mit dem Ring verbundenen Mikrokabels aufweist.

7. Sonde nach Anspruch 1, bei der das mindestens eine Lumen zum Durchqueren der Elektrode eine kreisförmige Öffnung mit einem Durchmesser größer als der Durchmesser des nicht mit dem Ring verbundenen Mikrokabels aufweist.

8. Sonde nach Anspruch 7, die eine zusätzliche isolierende Schutzhülle aufweist, die in das mindestens eine Lumen zum Durchqueren der Elektrode eingefügt ist.

9. Sonde nach Anspruch 1, bei der die kreisförmige Öffnung des mindestens einen Lumens zum Durchqueren der Elektrode in einem zentralen Bereich des Rings einen Durchmesser gleich dem Durchmesser der kreisförmigen Öffnung des Verbindungslumens aufweist.

10. Sonde nach Anspruch 1, bei der das mindestens eine Lumen zum Durchqueren der Elektrode geeignet ist, eine Mehrzahl von Mikrokabeln, die nicht mit dem Ring verbunden sind, aufzunehmen.

11. Sonde nach Anspruch 1, die mindestens einen Bereich (144) zum vorübergehenden Befestigen zwischen dem Verbindungsschieber und dem Ring (10) aufweist.

12. Sonde nach Anspruch 1, die am Umfang des Rings mindestens einen Bereich (18a, 18b, 18c) mit nicht durchgehenden Längsrillen (181) aufweist.

13. Sonde nach Anspruch 12, bei der der Radius der Längsrillen mindestens 5 µm beträgt.

14. Sonde nach Anspruch 1, bei der die Sonde mit einer kollektiven Außenhülle (30) aus wärmeschrumpfendem isolierendem Material abgedeckt ist, wobei Fenster (31) in dieser Hülle um Oberflächen (18a, 18b, 18c) zur Detektion/Stimulation des Rings ausgeschnitten sind.

15. Sonde nach Anspruch 14, bei der die Detektions-/Stimulationsoberflächen Bereiche sind, die Längsrillen (181) tragen.

16. Sonde nach Anspruch 1, bei der eine biegsame Klebstoffdichtung (40) an den Enden des Rings aufgebracht ist.

17. Sonde nach Anspruch 16, bei der der biegsame Klebstoff ein Polyurethan ist.

18. Sonde nach Anspruch 1, bei der eine Abfasung (16a, 16b) an jedem Ende des Rings eingerichtet ist.

19. Sonde nach Anspruch 1, bei der eine Stirnsenkung (17) an jedem Ende des Rings eingerichtet ist.

20. Sonde nach Anspruch 19, bei der die Stirnsenkung tangierend zu den Lumen des Rings ist.

21. Sonde nach Anspruch 1, bei der die Mittel zum elektrischen Verbinden einen Verbindungsstempel (14), der in dem Ring ausgeschnitten ist, aufweisen.

22. Sonde nach Anspruch 1, bei der die Mittel zum elektrischen Verbinden ein Perforierungsorgan (14') das mit dem Ring in ein und demselben Prägeabschnitt kombiniert ist, aufweisen, wobei das Perforierungsorgan und der Ring aus zwei Werkstoffen mit unterschiedlicher Steifigkeit bestehen.

23. Sonde nach Anspruch 1, bei der die Mittel zum elektrischen Verbinden ein Element (14"; 14"') zum alleinigen beweglichen Verbinden des Typs Gabel aufweisen.

24. Sonde nach Anspruch 23, bei der das Verbindungselement (14"') ein System zum Anclipsen in dem Verbindungslumen aufweist.

## Claims

1. A multipolar detection/stimulation lead intended to be implanted in a venous, arterial or lymphatic system, comprising:
- at least two micro-cables (21, 22a, 22b) each consisted of a solid central conductor (23) intended to be connected to a generator of an active implantable medical device, and of a sheath (24) made of an insulating polymer material surrounding the central conductor over the periphery and the length thereof;
- at least one detection/stimulation electrode intended to be electrically connected to a central conductor (23) of a micro-cable (21) and to come into contact with a wall of a target-vessel of said venous, arterial or lymphatic system ; and
- means for electrical connection between the electrode and a central conductor,
and wherein:
- the external diameter of the probe is at most equal to 2 French (0,66 mm); and
- said at least one electrode comprises a cylindrical metal ring (10) comprising a longitudinal electrical-connection hole (11), adapted to receive a micro-cable (21) intended to be connected to the ring, and at least one longitudinal electrode-through hole (12a, 12b),
**characterized in that**:
- the longitudinal electrode-through hole (12a, 12b) is adapted to receive at least one micro-cable (22a, 22b), not connected to the ring; and
- said at least one electrode also comprises means for electrical connection by piercing comprising at least one guiding tunnel (13a, 13b) transverse to the ring, arranged between the periphery of the ring and the electrical-connection hole, and an electrical-connection slide (14a, 14b) adapted to slide in the guiding tunnel and provided with an internal end (141a, 141b) shaped so as to pierce the sheath (24) of the micro-cable present in the connection hole so as to create an electrical contact between the ring and the central conductor of this micro-cable via the connection slide mounted and kept tight in the guiding tunnel.

2. The probe of claim 1, wherein the sliding axis (AA) of the connection slide passes by the centre of the connection hole.

3. The probe of claim 1, comprising two opposite connection slides.

4. The probe of claim 1, wherein the connection slide, in piercing position, is fully retracted into the guiding tunnel.

5. The probe of claim 1, wherein the internal end of the connection slide has a protruding shape.

6. The probe of claim 1, wherein the connection hole has a circular opening of diameter equal to the diameter of the micro-cable connected to the ring.

7. The probe of claim 1, wherein said at least one electrode-through hole has a circular opening of diameter higher than the diameter of the micro-cable non-connected to the ring.

8. The probe of claim 7, comprising an additional protective insulating sheath inserted into said at least one electrode-through hole.

9. The probe of claim 1, wherein the circular opening of said at least one electrode-through hole has, in a central area of the ring, a diameter equal to the diameter of the circular opening of the connection hole.

10. The probe of claim 1, wherein said at least one electrode-through hole is adapted to receive a plurality of micro-cables not connected to the ring.

11. The probe of claim 1, comprising at least one area (144) of temporary fastening between the connection slide and the ring (10).

12. The probe of claim 1, comprising, at the periphery of the ring, at least one area (18a, 18b, 18c) of non-through longitudinal grooves (181).

13. The probe of claim 12, wherein the radius of the longitudinal grooves is at least equal to 5 µm.

14. The probe of claim 1, wherein said probe is covered with a collective external sheath (30) made of a heat-shrinkable insulating material, windows (31) being cut in this sheath around detection/stimulation surfaces (18a, 18b, 18c) of the ring.

15. The probe of claim 14, wherein the detection / stimulation surfaces are areas carrying longitudinal grooves (181).

16. The probe of claim 1, wherein a flexible adhesive joint (40) is applied at the ends of the ring.

17. The probe of claim 16, wherein the flexible adhesive is polyurethane.

18. The probe of claim 1, wherein a chamfer (16a, 16b) is formed at each end of the ring.

19. The probe of claim 1, wherein a spot facing (17) is formed at each end of the ring.

20. The probe of claim 19, wherein the spot facing is tangent to the holes of the ring.

21. The probe of claim 1, wherein the electrical-connection means comprises a connection punch (14) cut into the ring.

22. The probe of claim 1, wherein the electrical-connection means comprise a piercing member (14') combined to the ring in a same punching section, the piercing member and the ring being consisted of two materials of different stiffness.

23. The probe of claim 1, wherein the electrical-connection means comprise a single, mobile, fork-type connection element (14"; 14"') .

24. The probe of claim 23, wherein the connection element (14"') includes a system for clipping into the connection hole.
